# EUROPÄISCHE PATENTANMELDUNG

(11) **EP 1 167 433 A1**
(43) Veröffentlichungstag der Anmeldung: **02.01.2002**
(21) Anmeldenummer: 01108690.7
(22) Anmeldetag: 06.04.2001
(51) Int. Cl.: C08L 1/00, C08L 33/00, A61K 9/20, C11D 17/00

(54) **Mit unlöslichem Sprengmittel coprozessiertes Polysaccharidprodukt, Herstellungsverfahren und Verwendung davon**

(30) Priorität: 19.06.2000 CH 12012000
(71) Anmelder: MIFA AG FRENKENDORF, CH-4402 Frenkendorf (CH)
(72) Erfinder: Bauer, Kurt, 79112 Freiberg-Tiengen (DE); Kleeli, Karin, 8121 Benglen (CH)
(74) Vertreter: Köver, François

(57) **Zusammenfassung**

Das Polysaccharidprodukt ist unter pulverförmiger Cellulose, mikrokristalliner Cellulose und Gemischen davon ausgewählt. Das unlösliche Sprengmittel ist ein unter Polyacrylsäure-Monopolymerisat, Polyacrylamid-Monopolymerisat, Polyacrylsäure-Polyacrylamid-Copolymerisat und Gemischen davon ausgewähltes Polyacrylprodukt. Vorzugsweise hat das Polysaccharidprodukt 1 bis 60 Gew.-% unlösliches Polyacrylprodukt-Sprengmittel und 40 bis 9° Gew.-% Cellulose, einen Restfeuchtegehalt von 1,5 bis 15%, und eine mittlere Korngrösse von 10 bis 2000 µm.

Das Polysaccharidprodukt kann durch mehrstufige Verfahren hergestellt werden, bei denen eine feuchte oder trockene Verdichtung angewendet werden kann.

Bei der Herstellung von Tabletten bringt das Polysaccharidprodukt eine sehr gute Tablettierbarkeit, es kann daher als Tablettensprengmittel eingesetzt werden.

## Beschreibung

Die Erfindung betrifft ein mit unlöslichem Sprengmittel coprozessiertes Polysaccharidprodukt, ein Herstellungsverfahren dazu und eine Verwendung davon.

Als Sprengmittel, Zerfallsbeschleuniger und/oder Zerfallhilfsmittel werden Hilfsstoffe bezeichnet, die für den raschen Zerfall von technischen oder pharmazeutischen Tabletten in Wasser, wässrigen Systemen oder Magensaft und für die rasche Freisetzung der in den Tabletten inkorporierten Wirkstoffe in fein dispergierter und aktiver Form sorgen - bei pharmazeutischen Tabletten soll diese aktive Form zudem eine gut resorbierbare Form sein. Besonders schnell wirkende Sprengmittel werden als Supersprengmittel bezeichnet. Es handelt sich bei all diesen Hilfsstoffen um Substanzen, welche die Porosität von Komprimaten erhöhen und ein grosses Adsorptionsvermögen für Wasser besitzen. Beispiele für bekannte Sprengmittel sind Stärke, Cellulosederivate, Alginsäuren, Dextrane und quervernetzte Polyvinylpyrrolidone sowie gasentwickelnde Substanzgemische wie z.B. Natriumhydrogencarbonat und Zitronen- oder Weinsäure (Brausemischungen). Beispiele für bekannte Supersprengmittel sind Carboxymethylstärke, unlösliche Carboxymethylcellulosen, unlösliches Polyvinylpyrrolidon, sowie Polyacryl-Supersprengmittel, umfassend unlösliche Polyacrylsäuren, unlösliche Polyacrylamide, unlösliche Polyacrylsäuren/Polyacrylamid-Copolymerisate und Gemische davon.

Die gängigen Sprengmittel, vor allem Stärke, aber auch die Supersprengmittel, sind allerdings mit Nachteilen behaftet. Stärkekörner allein sind elastisch und deshalb schlecht plastisch verformbar, also schlecht tablettierbar, und sie sind deshalb eher als Zerfallsbeschleuniger zu verwenden. Die vorerwähnten unlöslichen Supersprengmittel sind besser komprimierbar. Demgegenüber sind Celluloseprodukte plastischer und deshalb gut tablettierbar.

Der Erfindung liegt deshalb die Aufgabe zugrunde, mit Hilfe von bekannten stark quellenden Supersprengmitteln Tabletten bereitzustellen, die beim Einbringen in Wasser oder wässrige Systeme in Sekundenschnelle quellen, zerfallen und sich dispergieren. Daneben sollen die Tabletten, beim Einbringen in Wasser oder wässrige Systeme, wahlweise je nach dem Vermahlungsgrad der Produkte in gröbere, feine und feinste bis kolloidartige Dispersionen zerfallen. Weiterhin sollen Dispersions- bzw. Suspensionsstabilisatoren bereitgestellt werden, die bei der Herstellung von Kosmetika und von technischen Produkten, die sich sehr rasch dispergieren sollen, verwendbar sind.

Diese Aufgabe wird erfindungsgemäss gelöst durch ein mit unlöslichem Sprengmittel coprozessiertes Polysaccharidprodukt, das unter pulverförmiger Cellulose, mikrokristalliner Cellulose und Gemischen davon ausgewählt ist, wobei das unlösliche Sprengmittel ein unter unlöslichem Polyacrylsäure-Monopolymerisat, unlöslichem Polyacrylamid-Monopolymerisat, unlöslichem Polyacrylsäure-Polyacrylamid-Copolymerisat und Gemischen davon ausgewähltes unlösliches Polyacrylprodukt ist.

Pulverförmige Cellulose und mikrokristalline Cellulose (MCC) sowie deren Verwendung in Tabletten sind beim Stand der Technik wohlbekannt, vgl. beispielsweise in "Römpp Chemie Lexikon", 9. Auflage, Seite 615, Georg Thieme Verlag Stuttgart (1989). Die Herstellung von pulverförmigen Cellulose oder von mikrokristalliner Cellulose aus Zellstoff wird beispielsweise von B. Philipp und H.H. Steege in "Wissenschaft und Fortschritt" 25 (3), 126-131 (1975) beschrieben.

Für die Zwecke der Erfindung können als unlösliches Polyacrylprodukt alle beliebigen im Handel erhältlichen Polyacrylsäure-Monopolymerisate, Polyacrylamid-Monopolymerisate, Polyacrylsäure-Polyacrylamid-Copolymerisate sowie Gemische davon verwendet werden, soweit sie aufgrund ihres Vernetzungsgrades und/oder eines entsprechend hohen Polymerisationsgrades unlöslich aber in Wasser sehr stark quellfähig sind.

Geeignete unlösliche Polymerprodukte dieser Art werden beispielsweise in Firmenprospekten der BASF AG, D-67056 Ludwigshafen/Rhein und der Chemischen Fabrik Stockhausen GmbH, D-47805 Krefeld beschrieben. Bevorzugte unlösliche oder vernetzte Polymerprodukte dieser Art sind beispielsweise die im Handel erhältlichen hochmolekularen unlöslichen Sorten Hysorb® der BASF AG und Stockosorb® der Chemischen Fabrik Stockhausen.

Bei den erfindungsgemäss mit unlöslichem Polyacrylprodukt-Sprengmittel coprozessierten Polysaccharidprodukten, insbesondere bei Tabletten, wirkt die Cellulose beim Einbringen der Tablette in Wasser oder wässrige Systeme als Dochtsubstanz, die ausreichende Wassermengen mit ausreichender Geschwindigkeit ansaugt, damit das Sprengmittel seine Fähigkeiten voll entfalten kann. Die Cellulose quillt selber auch sehr gut, ohne jedoch dabei zu zerfallen. Die erfindungsgemässen Produkte vereinigen somit die Vorteile der guten Tablettierbarkeit und der starken Quellung und schnellen Zerfallbarkeit beim Einbringen in Wasser oder wasserhaltige Flüssigkeiten. Die gute Tablettierbarkeit der erfindungsgemässen Produkte ermöglicht deren erfolgreiche Verwendung als Tablettensprengmittel, -zerfallsbeschleuniger bzw. -zerfallshilfsmittel, wobei die schnelle und starke Quellung durch eine optimale, möglichst hohe Verdichtung des Zweikomponentensystems bei einer trockenen oder feuchten Coprozessierung noch erheblich verbessert wird.

Ein erfindungsgemäss mit unlöslichem Polyacrylprodukt-Sprengmittel coprozessiertes Polysaccharidprodukt hat vorzugsweise folgende Kenndaten:
Bei den gemahlenen, coprozessierten Zubereitungen des erfindungsgemässen Produkts liegen die Schüttdichten bei Verwendung von Cellulosepulver bei 0,120-0,600 g/ml, vorzugsweise bei 0,250-0,500 g/ml. Bei Verwendung von mikrokristalliner Cellulose (MCC) liegen die Schüttdichten bei 0,300-0,750 g/ml, vorzugsweise bei 0,350-0,600 g/ml. Die Schüttdichte des erfindungsgemässen Produkts liegt vorzugsweise höher als die Schüttdichte des Ausgangspulvergemisches.

Insbesondere hat das erfindungsgemässe Produkt einen Restfeuchtegehalt von 1,5 bis 15%, vorzugsweise von weniger als 10%, noch bevorzugter von 4 bis 7%.

Der Gehalt der einzelnen Komponenten im erfindungsgemässen Produkt kann innerhalb weiter Grenzen variieren, beispielsweise von 1 bis 60 Gew.-% unlöslichem Polyacrylprodukt-Sprengmittel und 40 bis 99 Gew.-% Cellulose. Bevorzugt wird ein Gehalt von 3 bis 60 Gew.-% unlöslichem Polyacrylprodukt-Sprengmittel und 40 bis 97 Gew.-% Cellulose. Noch mehr bevorzugt wird ein Gehalt von 5 bis 30 Gew.-% unlöslichem Polyacrylprodukt-Sprengmittel und 70 bis 95 Gew.-% Cellulose.

Wahlweise können dem erfindungsgemässen Produkt noch geringe Mengen weiterer Zerfallshilfsstoffe, beispielsweise diverse Stärken, Brausemischungen z.B. aus Natriumcarbonat und Natriumhydrogensulfat, usw. zugesetzt werden, wobei diese Mengen durch entsprechende Abzüge bei der Menge Cellulose ausgeglichen d.h. kompensiert werden.

Auch die Korngrösse des erfindungsgemässen Produkts kann innerhalb weiter Grenzen variieren, wie beispielsweise von 10 bis 2000 µm, vorzugsweise von 50 bis 1000 µm.

Das erfindungsgemässe Produkt kann durch Coprozessierung einer wie oben definierten Cellulose mit einem wie oben definierten unlöslichen Sprengmittel durch feuchtes oder trockenes Verdichten unter Druck erhalten werden. Unter dem Begriff "Coprozessierung" wird hier ein trockenes Verdichten z.B. zwischen gegenläufigen Kompaktierwalzen bei Drücken von 20-60 kN, vorzugsweise von 30-50 kN, oder ein feuchtes Verdichten nach Zusatz von Wasser, durch Kneten oder Pressen von feuchtplastischen Massen durch ein Sieb, eine Lochscheibe oder über einen Extruder und abschliessendem Trocknen verstanden.

Durch die Erfindung wird weiterhin ein Verfahren zur Herstellung des erfindungsgemässen Produkts bereitgestellt.

Bei einer ersten alternativen Ausführungsform ist dieses Verfahren gekennzeichnet durch:
(a) Suspendieren eines feuchten bzw. nicht getrockneten, nicht verhornten Polysaccharidprodukts, das unter pulverförmiger Cellulose, mikrokristalliner Cellulose und Gemischen davon ausgewählt ist, in einem wässrigen Medium zu einer homogenen Dispersion;
(b) Vermengen der so erhaltenen Dispersion nach deren Abkühlen unter 40°C mit einem unlöslichen Sprengmittel, das ein unter unlöslichem Polyacrylsäure-Monopolymerisat, unlöslichem Polyacrylamid-Monopolymerisat, unlöslichem Polyacrylsäure-Polyacrylamid-Copolymerisat und Gemischen davon ausgewähltes unlösliches Polyacrylprodukt ist;
(c) gegebenenfalls Vortrocknen des so erhaltenen vermengten Produkts;
(d) Verdichten des so erhaltenen vermengten und gegebenenfalls vorgetrockneten Produkts unter Druck;
(e) Trocknen des so erhaltenen verdichteten Produkts bei einer Temperatur von 20 bis 90°C; und
(f) Vermahlen des so erhaltenen getrockneten Produkts auf eine mittlere Korngrösse von 10 bis 2000 µm.

Bevorzugte Weiterbildungen des erfindungsgemässen Verfahrens in seiner ersten alternativen Ausführungsform sind: Das Dispersionsmedium kann ein heisses wässriges Medium sein. Die im wässrigen Medium vorliegende homogene Dispersion kann eine Suspension oder eine pastenförmige vorverdichtete Masse sein, und sie kann weitere Zerfallshilfsstoffe enthalten. Das Verdichten unter Druck kann ein Passieren, insbesondere ein Pressen des in Stufe (b) bzw. (c) erhaltenen und gegebenenfalls vorverdichteten feuchtplastischen Produkts durch ein Sieb, eine Lochscheibe oder einen Extruder zwecks Kompaktierung und Aggregatbildung sein. Die mittlere Korngrösse des vermahlenen Produkts kann von 50 bis 1000 um betragen.

Als Cellulose-Ausgangsprodukt wird vorzugsweise eine nicht getrocknete mikrokristalline oder pulverförmige Cellulose verwendet. Pulverförmige Cellulose wird beispielsweise in an sich bekannter Weise aus Zellstoff oder faseriger Rohcellulose hergestellt. Mikrokristalline Cellulose wird beispielsweise aus Zellstoffpulpe durch Erhitzen mit einer 3-bis 5%-igen starken Mineralsäure, beispielsweise Salz- oder Schwefelsäure, hergestellt. Für die Zwecke der Erfindung besonders geeignete, nicht getrocknete mikrokristalline oder pulverförmige Cellulose ist praktisch ein Zwischen- oder Vorprodukt der gängigen Herstellung von reiner pulverförmiger oder mikrokristalliner Cellulose, vgl. beispielsweise B. Philipp und H.H. Steege in "Wissenschaft und Fortschritt" 25 (3), 126-131 (1975).

Unter "nicht getrocknet" werden, mit Bezug auf Cellulose, Vorprodukte verstanden, die aus dem Herstellungsgang, nach der Reinigung, aber vor der Endtrocknung gewonnen werden, z.B. bei der oben beschriebenen Behandlung von Zellstoffpulpe mit Mineralsäure nach dem Entfernen der Säure und der erforderlichen Reinigung der hierbei entstandenen mikrokristallinen Cellulose. Das Cellulose:Wasser-Verhältnis kann 1,0:0,15 Teile bis 1,0:30,0 Teile, vorzugsweise 1,0:1,0 Teile bis 1,0:10,0 Teile betragen. Bei einer Trocknung des Produkts zu einem Feuchtigkeitsgehalt von weniger als 1,5 Teile Wasser pro 10,0 Teile Cellulose kann es vorkommen, dass die Oberfläche der Cellulose zu trocken wird und dass das Produkt in unerwünschter Weise "verhornt". In solchen Fällen sind die Polysaccharidprodukte nicht mehr optimal für die oben angegebenen Zwecke geeignet, da verhornte Bereiche nur mehr stark eingeschränkt hydratisieren können. Andererseits führen zu grosse Flüssigkeits- bzw. Wassermengen dazu, dass die Produkte nach der Zugabe von unlöslichem Polyacrylprodukt und gegebenenfalls weiterer Zerfallshilfsstoffe nicht ausreichend feuchtplastisch, sondern zu flüssig sind, so dass eine Obergrenze von 30,0 Teile, vorzugsweise von 25,0 Teile Wasser pro 1,0 Teil Cellulose bevorzugt wird. Ausserdem ist das Trocknen von hohen Feuchtigkeitsanteilen nicht wirtschaftlich. Relativ hohe Mengen von Wasser sind zu einer ausreichenden Verdichtung erforderlich, aber zu hohe Mengen können entsprechend den in der nächsten Stufe (b) verwendeten Mengen des unlöslichen Polyacrylprodukts je nach dessen Vermahlungsgrad die Gemische dann zu feucht werden lassen, so dass sie nicht mehr feuchtplastisch verformbar bzw. verdichtbar sind und nur noch schwer und langwierig getrocknet werden können.

Somit wird in der ersten Stufe (a) dieses Verfahrens ein feuchtes bzw. nicht getrocknetes, nicht verhorntes Polysaccharidprodukt, das unter pulverförmiger Cellulose, mikrokristalliner Cellulose und Gemischen davon ausgewählt ist, in einem wässrigen, zur besseren Hydratisierung vorzugsweise warmen bis heissen Medium zu einer homogenen Dispersion suspendiert.

Als wässriges Medium kann beispielsweise Wasser, wie Leitungswasser, verwendet werden, das gegebenenfalls bis zum Sieden aufgeheizt werden kann. Die Temperatur beim Suspendierungsvorgang beträgt 40 bis 100°C, vorzugsweise 80 bis 100°C. Die Suspendierung kann in an sich bekannter Weise in einer geeigneten Vorrichtung, wie einem Schnellmischer oder Dispergator, verwendet werden. Geeignete Vorrichtungen sind im Handel erhältlich und werden beispielsweise in Bauer, Frömming, Führer: "Pharmazeutische Technologie", Wissenschaftl. Verlagsgesellschaft, Stuttgart, 6. Auflage, 1999, Seiten 134-136 beschrieben. Bei dieser Befeuchtung, d.h. bei diesem Suspensionsvorgang, kann gegebenenfalls schon eine Vorverdichtung erzielt werden, z.B. durch stärkere Hydratation bei Verwendung von heissem Wasser. Weiterhin kann durch Kneten, beispielsweise im Z-Kneter mit kräftigen Mischarmen, im Gegensatz zu einfach gerührten bzw. gemischten Massen eine stärkere Verdichtung oder Vorverdichtung erreicht werden. Die so erhaltene Dispersion hat beispielsweise einen Feststoffgehalt von 10 bis 90 Gew.-%, vorzugsweise von 20 bis 60 Gew.-%.

In der nächsten Stufe (b) dieses Verfahrens wird die in Stufe (a) erhaltene Suspension nach Abkühlung unter 40°C mit einem unlöslichen Sprengmittel vermengt, das ein unter unlöslichem Polyacrylsäure-Monopolymerisat, unlöslichem Polyacrylamid-Monopolymerisat, unlöslichem Polyacrylsäure-Polyacrylamid-Copolymerisat und Gemischen davon ausgewähltes unlösliches Polyacrylprodukt ist. Wie bereits oben ausgeführt, können in dieser Stufe alle beliebigen im Handel erhältlichen Polyacrylsäure-Monopolymerisate, Polyacrylamid-Monopolymerisate, Polyacrylsäure-Polyacrylamid-Copolymerisate sowie Gemische davon verwendet werden, soweit sie aufgrund ihres Vernetzungsgrades unlöslich sind.

Die Menge der in dieser Stufe zugegebenen unlöslichen Polyacrylprodukts wird so bemessen, dass das Endprodukt 3,0 bis 60 Gew.-%, vorzugsweise 5 bis 40 Gew.-% unlösliches Polyacrylprodukt enthält.

Die Vermengung der Ausgangsprodukte kann vorzugsweise durch Verkneten geschehen. Geeignete Kneter werden beispielsweise in Bauer, Frömming, Führer: "Pharmazeutische Technologie", Wissenschaftl. Verlagsgesellschaft, Stuttgart, 6. Auflage, 1999, Seiten 134-136 beschrieben.

Die Temperatur bei der Vermengung bzw. Verknetung beträgt 50 bis 30°C, vorzugsweise 40 bis 20°C. Vorzugsweise erfolgt die Verknetung bei Raumtemperatur, damit gegebenenfalls wärmeempfindliche Produkte nicht nachteilig beeinflusst werden. Eine Verknetung kann daneben auch grundsätzlich dann vorgenommen werden, um eine Verdichtung zu erzielen.

In der nächsten Stufe (c) dieses Verfahrens wird das in Stufe (b) erhaltene Gemisch gegebenenfalls zu einem Restfeuchtegehalt von 1,5 bis 15 Gew.-%, vorzugsweise von 4 bis 7 Gew.-% vorgetrocknet. Als Trockner können übliche Trocknungsvorrichtungen wie Trockenschränke und Wirbelschichttrockner eingesetzt werden. Geeignete Trockner werden beispielsweise in Bauer, Frömming, Führer: "Pharmazeutische Technologie", Wissenschaftl. Verlagsgesellschaft, Stuttgart, 6. Auflage, 1999, Seiten 123-130 beschrieben. Bevorzugt wird die Vortrocknung bei Temperaturen von 30 bis 70°C. insbesondere 50 bis 60°C durchgeführt.

In der nächsten Stufe (d) dieses Verfahrens wird das in Stufe (b) bzw. (c) erhaltene feuchtplastische, durch das Befeuchten bereits vorverdichtete Produkt möglichst weitgehend verdichtet. Dazu kann das Produkt beispielsweise durch ein Sieb geschlagen bzw. gepresst werden, wobei übliche Siebe in Betracht kommen, beispielsweise solche, die in Bauer, Frömming, Führer: "Pharmazeutische Technologie", Wissenschaftl. Verlagsgesellschaft, Stuttgart, 6. Auflage, 1999, Seite 108 (Siebe) bzw. Seite 308 (Extrudergranulierung) beschrieben werden. Geeignete Siebe haben eine Maschenweite von 0,5 mm bis 5 mm und vorzugsweise von 1 bis 2 mm. Das Durchschlagen bzw. Durchpressen des Produkts durch Siebe, Lochscheiben oder Extruder erfolgt im übrigen auf übliche Weise und bei üblichen Bedingungen wie Normaltemperatur. Das Sieben kann bei Normaldruck oder gegebenenfalls unter Anwendung von Überdruck erfolgen.

In der nächsten Stufe (e) dieses Verfahrens wird die verdichtete Masse bei einer Temperatur von 20 bis 90°C, vorzugsweise von 50 bis 80°C getrocknet. Die Trocknung erfolgt in der gleichen Weise wie die oben im Zusammenhang mit Stufe (c) beschriebene Vortrocknung. Auch hier können übliche Trockner, wie Trockenschränke und Wirbelschichttrockner, eingesetzt werden.

In der letzten Stufe (f) dieses Verfahrens wird schliesslich das so erhaltene Produkt auf eine mittlere Korngrösse von 10 bis 2000 µm, vorzugsweise von 50 bis 1000 µm vermahlt. Für den Vermahlungsvorgang können alle beliebigen geeigneten Mühlen, wie beispielsweise Kugelmühlen und dergleichen verwendet werden. Geeignete Vorrichtungen sind beispielsweise in Bauer, Frömming, Führer: "Pharmazeutische Technologie", Wissenschaftl. Verlagsgesellschaft, Stuttgart, 6. Auflage, 1999, Seiten 104-107 beschrieben.

Bei einer zweiten alternativen Ausführungsform ist das erfindungsgemässe Verfahren gekennzeichnet durch:
(g) Vermischen eines pulverförmigen Polysaccharidprodukts, das unter pulverförmiger Cellulose, mikrokristalliner Cellulose und Gemischen davon ausgewählt ist, mit einem pulverförmigen unlöslichen Sprengmittel, das ein unter unlöslichem Polyacrylsäure-Monopolymerisat, unlöslichem Polyacrylamid-Monopolymerisat, unlöslichem Polyacrylsäure-Polyacrylamid-Copolymerisat und Gemischen davon ausgewähltes unlösliches Polyacrylprodukt ist;
(h) Sieben des so erhaltenen Gemisches durch ein feines Sieb;
(i) Verdichten des so erhaltenen gesiebten Produkts;
(j) Verkleinern des so erhaltenen verdichteten Produkts; und
(k) Vermahlen des so erhaltenen verkleinerten Produkts auf eine mittlere Korngrösse von 10 bis 2000 µm.

Bevorzugte Weiterbildungen des erfindungsgemässen Verfahrens in seiner ersten alternativen Ausführungsform sind:
Das Verdichten kann durch Kompaktieren zu Schülpen oder durch Brikettieren mit Kompaktierwalzen oder einer Exzenterpresse bei Drücken von 20-60 kN, vorzugsweise von 30-50 kN erfolgen.
Das Sieben kann durch ein Sieb mit einer Maschenweite von 1,2 mm erfolgen. Das Verkleinern kann ein Granulieren oder ein verkleinerndes Homogenisieren der Briketts oder Schülpen mit Stachelwalzen oder dergleichen oder ein Vermahlen mit einer geeigneten Mühle sein. Die mittlere Korngrösse des vermahlenen Produkts kann von 50 bis 1000 µm betragen.

Im Vergleich zur ersten zweiten alternativen Ausführungsform entfallen bei der zweiten alternativen Ausführungsform die Herstellungsschritte der Suspendierung (a), des Vortrocknens (c), das Pressen durch Siebe oder Extruder (d) und der Endtrocknung (e) der ersten Alternative.

Die Qualität der Verdichtung hängt von den Kompaktierdrucken ab, in zweiter Linie auch noch von den Restfeuchtigkeitsgehalten des Gemisches aus dem Schritt (g), die zwischen 3 und 40 Gew.-%, vorzugsweise zwischen 5 und 25 Gew.-% liegen kann. Endprodukte mit höheren Feuchtigkeitsgehalten müssen gegebenenfalls nachgetrocknet werden, bis sie Restfeuchtigkeiten von 1,5-15 Gew.-%, vorzugsweise von 4 - 7 Gew.-% aufweisen. Die Trockenverdichtungs- bzw. Trockengranulierverfahren werden beispielsweise in Bauer, Frömming, Führer: "Pharmazeutische Technologie", Wissenschaftl. Verlagsgesellschaft, Stuttgart, 6. Auflage, 1999, Seiten 306, 308 und 310 beschrieben.

Die so hergestellten, erfindungsgemässen, mit unlöslichem Polyacrylprodukt-Sprengmittel coprozessierten Polysaccharidprodukte zeichnen sich durch ausserordentlich schnelle und starke Quellung beim Einbringen in Wasser oder wasserhaltige Flüssigkeiten sowie durch sehr gute Tablettierbarkeit aus. Sie können daher als äusserst wirksame Sprengmittel, Zerfallsbeschleuniger und/oder Zerfallhilfsmittel für Tabletten eingesetzt werden.

Bei den als Sprengmittel für Tabletten hochwirksamen erfindungsgemässen Produkten ist als Vorteil der durch die innige Mischung und Coprozessierung der Bestandteile (Polysaccharidprodukte, unlösliche Polyacrylprodukte, gegebenenfalls weitere Zerfallshilfsstoffe) erreichte innige Kontakt und gute Zusammenhalt hervorzuheben, der für die optimale Sprengwirkung entscheidend ist und auch nach dem Einarbeiten von Wirkstoffen (Arzneimitteln, Tensiden und dergleichen) und anderen Komponenten einer Tablettenrezeptur erhalten bleibt. Ein weiterer Vorteil der intensiven Coprozessierung der Bestandteile ist, dass auch mechanisch feste, stark komprimierte, wenig poröse Tabletten damit herstellbar sind.

Unter Verwendung der erfindungsgemässen Produkte können Dispersionstabletten hergestellt werden, die beim Einbringen in Wasser sofort, also in Sekundenschnelle, quellen, zerfallen und sich dispergieren oder lösen. Je nach Vermahlungsgrad des erfindungsgemässen Produkts lassen sich aus den damit hergestellten Tabletten gröbere, feine und feinste bis kolloidartige Dispersionen erzeugen. Neben der Verwendbarkeit als Tablettenzerfallsbeschleuniger ist natürlich ein Einsatz als Dispersions- oder Suspensionsstabilisator bei der direkten Herstellung von flüssigen und halbfesten Zubereitungen wie Sirupen, Lotionen, Säften, Gelen, Hydrosolen und Hydrogelen, Salbenzubereitungen und Pasten denkbar und möglich. Einsatzmöglichkeiten gibt es überall, wo schnelle und feinste Dispergierungen erreicht und stabilisiert werden sollen. Die Anwendung umfasst, ohne Einschränkung darauf, Pharmazeutika, Kosmetika und technische Formulierungen.

Die Erfindung wird in den nachstehenden Beispielen näher erläutert.

### HERSTELLUNGSBEISPIELE

### Beispiel 1

1000,0 kg feuchtes, nicht verhorntes Cellulosepulver mit einem Wassergehalt von ca. 20% wird möglichst bald nach der Herstellung durch Zugabe von 2500,0 kg Wasser resuspendiert. Die Suspension wird mit einem Schnellmischer oder Dispergator homogen dispergiert, und anschliessend werden 160,0 kg handelsübliches unlösliches Polyacrylprodukt wie Hysorb® der BASF AG und Stockosorb® der Chemischen Fabrik Stockhausen eingeknetet. Die feuchtplastische Masse wird durch einen Extruder mit 1 mm Maschenweite bzw. Lochdurchmesser geschlagen und in einem geeigneten Trockner bei 60°C getrocknet. Das fertig coprozessierte Produkt hat einen Restfeuchtegehalt von 5 bis 8 Gew.-% und mittlere Partikelgrössen von 0,25 bis 0,8 mm.

### Beispiel 2

2250,0 kg feuchter Kuchen mit einem Verhältnis (mikrokristalline Cellulose) : (Wasser) = 40:60 wird mit der gleichen Menge Wasser resuspendiert und homogenisiert. Anschliessend werden 100,0 kg handelsübliches unlösliches Polyacrylprodukt wie Hysorb® der BASF AG und Stockosorb® der Chemischen Fabrik Stockhausen eingeknetet, die feuchtplastische Masse durch ein 1,5-mm-Sieb geschlagen und in einem Wirbelschichttrockner (Zulufttemperatur 100-120°C) bis zu einer Restfeuchte von ca. 5% getrocknet (Ausgangslufttemperatur 35-45°C).

### Beispiel 3

2000,0 kg feuchter Kuchen aus mikrokristalliner Cellulose und Wasser im Verhältnis 50:50 wird mit der doppelten Menge Wasser angeschlämmt bzw. resuspendiert und 5 min lang mit einem UltraTurrax homogenisiert. Anschliessend wird die homogene Mischung in einen Planetenmischer gegeben, und bei laufendem Rührwerk wird portionenweise 300,0 kg handelsübliches unlösliches Polyacrylprodukt wie Hysorb® der BASF AG und Stockosorb® der Chemischen Fabrik Stockhausen sowie 50,0 kg Maisstärke dazugeknetet und abschliessend 10 min gemischt. Die feuchtplastische Masse wird in dünnen Schichten und angemessenen Portionen entweder im Trockenschrank bei 50°C oder im Wirbelschicht-Trockner bei einer Zulufttemperatur von 80-90°C bis zur siebbaren Konsistenz vorgetrocknet. Dann wird sie durch ein 5-mm-Sieb geschlagen und im Wirbelschicht-Trockner bei einer Zulufttemperatur von 80° zu Ende getrocknet (Restfeuchte ca. 5%). Zuletzt wird die Masse durch ein 1,00mm-Sieb geschlagen und in einer geeigneten Mühle auf eine mittlere Korngrösse von 80 µm gemahlen.

### Beispiel 4

2000,0 g feuchtes, feingemahlenes Cellulosepulver (mittlere Partikelgrösse 50 µm, Feuchtigkeitsgehalt ca. 50%) wird mit 1500,0 g siedendem, deionisiertem Wasser in einem geeigneten Mischer homogen befeuchtet. Anschliessend werden 100,0 g handelsübliches unlösliches Polyacrylprodukt wie Hysorb® der BASF AG und Stockosorb® der Chemischen Fabrik Stockhausen eingeknetet, so dass eine feuchtplastische Masse entsteht, die mit möglichst hohem Druck durch einen Extruder gepresst wird, dessen Lochscheibe Bohrungen von 1 bis 3 mm, vorzugsweise von 1 bis 2 mm aufweist. Das so entstehende Extrudergranulat wird bei 80°C bis zu einer Restfeuchte von 4 bis 6 % getrocknet und anschliessend mit einer Rotor-Stator- oder Stift-Mühle auf eine mittlere Korngrösse von 0,2 mm vermahlen.

### Beispiel 5

9,0 kg feingemahlenes Cellulosepulver (mittlere Partikelgrösse 40 µm) und 1,0 kg unlösliches Polyacrylprodukt wie Hysorb® der BASF AG und Stockosorb® der Chemischen Fabrik Stockhausen werden homogen gemischt und durch ein 1-mm-Sieb geschlagen. Anschliessend wird diese Mischung mit Hilfe einer Kompaktierwalze bei Drucken von 30-45 kN brikettiert und durch ein 0,2-mm- oder 1-mm-Sieb geschlagen. Die Schüttdichte des so hergestellten Produkts liegt bei 0,400-0,450 g/ml.

### Beispiel 6

18,0 kg feingemahlenes Cellulosepulver (mittlere Partikelgrösse 70 bis 90 µm), 2,0 kg unlösliches Polyacrylprodukt wie Hysorb® der BASF AG und Stockosorb® der Chemischen Fabrik Stockhausen, 7,2 kg Natriumcarbonat und 9,6 kg Natriumhydrogensulfat werden homogen gemischt und durch ein 1-mm-Sieb geschlagen. Anschliessend wird diese Mischung mit Hilfe einer Kompaktierwalze bei Drucken von 30-45 kN brikettiert und durch ein 0,2-mm- oder 1-mm-Sieb geschlagen. Die Schüttdichte des so hergestellten Produkts liegt bei 0,400-0,500 g/ml.

### VERWENDUNGSBEISPIELE

### Beispiel 7

Unter Verwendung des erfindungsgemässen Polysaccharidprodukts, d.h. mit unlöslichem Polyacrylprodukt coprozessierter gemahlener Cellulose, wurden Tabletten in folgender Weise hergestellt: Es wurden 1000,0 g ASS (Acetylsalicylsäure) von mittlerer Korngrösse 0,2 mm mit 100,0 g des erfindungsgemässen Polysaccharidprodukts gemischt und auf einer üblichen Tablettenpresse zu Tabletten mit 11 mm Durchmesser und 550 mg Bruttogewicht verpresst. Die so hergestellten Tabletten wurden folgendem Zerfallstest in folgender Weise unterworfen:
a) Einfaches Einlegen der Tabletten in ein Becherglas mit ca. 200-250 ml reinem Wasser bei Raumtemperatur und Beobachtung des Zerfalls;
b) Einbringen jeweils einer Tablette, die in einem Drahtbügel eingeklemmt ist, in ein Becherglas mit 250 ml reinem Wasser bei Raumtemperatur und Bestimmung des Zeitraums bis zum vollständigen Zerfall. Die erhaltenen Ergebnisse sind in folgender Tabelle zusammengestellt:

| | | b) |
|---|---|---|
| Tablette mit 500 mg ASS + 50 mg des erfindungsgemässen Polysaccharidprodukts, coprozessiert nach Beispiel 1 | zerfällt sehr rasch | < 10 s |
| Tablette mit 500 mg ASS + 50 mg Crospovidone | zerfällt sehr rasch | 20-25 s |
| Tablette mit 500 mg ASS + 50 mg des erfindungsgemässen Polysaccharidprodukts, coprozessiert nach Beispiel 5 | zerfällt sehr rasch | < 10 s |
| Tablette mit 500 mg ASS + 50 mg Weizenstärke | quillt stark, aber zerfällt nicht | 35-45 s |

Aus der obigen Tabelle ergibt sich, dass die unter Verwendung der erfindungsgemässen Polysaccharidprodukte hergestellten Tabletten im Vergleich zu den Produkten mit herkömmlichen Sprengmitteln deutlich verbesserte Zerfallseigenschaften haben.

### Beispiel 8

Unter Verwendung des erfindungsgemässen Polysaccharidprodukts als Sprengmittel wurden Tabletten mit den nachstehend angegebenen Zusammensetzungen hergestellt:

| Für ein Wasserenthärtungsmittel: | |
|---|---|
| Trinatriumcitrat | 30,0 Gew.-% |
| Natriumcarbonat | 24,0 Gew.-% |
| Natriumdisilicat | 15,0 Gew.-% |
| Zeolith | 15,0 Gew.-% |
| Polycarboxylat | 4,0 Gew.-% |
| | |
| Sprengmittel | 12,0 Gew.-% |

| Für ein Waschmittel: | |
|---|---|
| Natriumperborat, Tetrahydrat | 22,0 Gew.-% |
| gNatriumcarbonat | 15,0 Gew.-% |
| Zeolith | 15,0 Gew.-% |
| Alkylbenzolsulfonsäure, Na-Salz | 13,0 Gew.-% |
| Silicate | 9,1 Gew.-% |
| Tetraacetylethylendiamin | 6,0 Gew.% |
| Polycarboxylat | 5,0 Gew.-% |
| Fettalkoholethoxylat 7EO | 2,0 Gew.-% |
| Enzyme | 2,0 Gew.-% |
| Entschäumer | 0,5 Gew.-% |
| Phosphonat | 0,4 Gew.-% |
| | |
| Sprengmittel | 10,0 Gew.-% |

| Für ein Bleichmittel: | |
|---|---|
| Natriumperborat, Monohydrat | 30,0 Gew.-% |
| Natriumhydrogencarbonat | 15,0 Gew.-% |
| Tetraacetylethylendiamin | 15,0 Gew.% |
| Natriumdisilicat | 15,0 Gew.-% |
| Natriumsulfat | 11,0 Gew.-% |
| Enzyme | 2,0 Gew.-% |
| | |
| Sprengmittel | 12,0 Gew.-% |

| Für ein Geschirrspülmittel: | |
|---|---|
| Natriumtripolyphosphat | 50,0 Gew.-% |
| Natriumcarbonat | 16,9 Gew.-% |
| Natriumperborat, Monohydrat | 10,0 Gew.-% |
| Natriumdisilicat | 10,0 Gew.-% |
| Enzyme | 2,5 Gew.-% |
| Tetraacetylethylendiamin | 2,0 Gew.% |
| Fettalkoholalkoxylat | 1,5 Gew.-% |
| Benzotriazol | 0,1 Gew.-% |
| | |
| Sprengmittel | 7,0 Gew.-% |

| Für ein Bademittel: | |
|---|---|
| Citronensäure | 30,0 Gew.-% |
| Natriumhydrogencarbonat | 20,0 Gew.-% |
| Natriumsulfat | 41,0 Gew.-% |
| Parfum | 3,5 Gew.-% |
| Farbmittel | 0,5 Gew.-% |
| | |
| Sprengmittel | 5,0 Gew.-% |

Tabletten dieser und ähnlicher Art haben sich bei den angegebenen wie auch bei ähnlichen Anwendungen gut bewährt, das heisst, sie zerfallen wie gewünscht rasch und vollständig.

## Patentansprüche

1. Mit unlöslichem Sprengmittel coprozessiertes Polysaccharidprodukt, **dadurch gekennzeichnet, dass** das Polysaccharidprodukt unter pulverförmiger Cellulose, mikrokristalliner Cellulose und Gemischen davon ausgewählt ist, und dass das unlösliche Sprengmittel ein unter unlöslichem Polyacrylsäure-Monopolymerisat, unlöslichem Polyacrylamid-Monopolymerisat, unlöslichem Polyacrylsäure-Polyacrylamid-Copolymerisat und Gemischen davon ausgewähltes unlösliches Polyacrylprodukt ist.

2. Polysaccharidprodukt nach Anspruch 1, **dadurch gekennzeichnet, dass** es einen Restfeuchtegehalt von 1,5 bis 15%, vorzugsweise von weniger als 10%, noch bevorzugter von 4 bis 7% hat.

3. Polysaccharidprodukt nach Anspruch 1, **dadurch gekennzeichnet, dass** es von 1 bis 60 Gew.-% unlösliches Polyacrylprodukt-Sprengmittel und 40 bis 99 Gew.-% Cellulose, vorzugsweise von 3 bis 60 Gew.-% unlösliches Polyacrylprodukt-Sprengmittel und 40 bis 97 Gew.-% Cellulose, noch mehr bevorzugt von 5 bis 30 Gew.-% unlösliches Polyacrylprodukt-Sprengmittel und 70 bis 95 Gew.-% Cellulose enthält.

4. Polysaccharidprodukt nach Anspruch 1, **dadurch gekennzeichnet, dass** es eine mittlere Korngrösse von 10 bis 2000 µm, vorzugsweise 10 bis 1000 µm hat.

5. Polysaccharidprodukt nach Anspruch 1, **dadurch gekennzeichnet, dass** das coprozessierte Gemisch von Polysaccharidprodukt und unlöslichem Polyacrylprodukt eine höhere Schüttdichte hat als dasselbe Gemisch vor dessen Coprozessierung.

6. Verfahren zur Herstellung eines Polysaccharidprodukts nach Anspruch 1, **gekennzeichnet durch**:
(a) Suspendieren eines feuchten bzw. nicht getrockneten, nicht verhornten Polysaccharidprodukts, das unter pulverförmiger Cellulose, mikrokristalliner Cellulose und Gemischen davon ausgewählt ist, in einem wässrigen Medium zu einer homogenen Dispersion;
(b) Vermengen der so erhaltenen Dispersion nach deren Abkühlen unter 40°C mit einem unlöslichen Sprengmittel, das ein unter unlöslichem Polyacrylsäure-Monopolymerisat, unlöslichem Polyacrylamid-Monopolymerisat, unlöslichem Polyacrylsäure-Polyacrylamid-Copolymerisat und Gemischen davon ausgewähltes unlösliches Polyacrylprodukt ist;
(c) gegebenenfalls Vortrocknen des so erhaltenen vermengten Produkts;
(d) Verdichten des so erhaltenen vermengten und gegebenenfalls vorgetrockneten Produkts unter Druck;
(e) Trocknen des so erhaltenen verdichteten Produkts bei einer Temperatur von 20 bis 90°C; und
(f) Vermahlen des so erhaltenen getrockneten Produkts auf eine mittlere Korngrösse von 10 bis 2000 µm.

7. Verfahren zur Herstellung eines Polysaccharidprodukts nach Anspruch 6, **dadurch gekennzeichnet, dass** das Vermengen und Verdichten durch Verkneten erfolgt.

8. Verfahren zur Herstellung eines Polysaccharidprodukts nach Anspruch 1, **gekennzeichnet durch**:
(g) Vermischen eines pulverförmigen Polysaccharidprodukts, das unter pulverförmiger Cellulose, mikrokristalliner Cellulose und Gemischen davon ausgewählt ist, mit einem pulverförmigen unlöslichen Sprengmittel, das ein unter unlöslichem Polyacrylsäure-Monopolymerisat, unlöslichem Polyacrylamid-Monopolymerisat, unlöslichem Polyacrylsäure-Polyacrylamid-Copolymerisat und Gemischen davon ausgewähltes unlösliches Polyacrylprodukt ist;
(h) Sieben des so erhaltenen Gemisches **durch** ein feines Sieb;
(i) Verdichten des so erhaltenen gesiebten Produkts;
(j) Verkleinern des so erhaltenen verdichteten Produkts; und
(k) Vermahlen des so erhaltenen verkleinerten Produkts auf eine mittlere Korngrösse von 10 bis 2000 um.

9. Verwendung des Polysaccharidprodukts nach einem der Ansprüche 1 bis 5 als Sprengmittel, Zerfallsbeschleuniger und/oder Zerfallhilfsmittel für Tabletten.

10. Verwendung des Polysaccharidprodukts nach einem der Ansprüche 1 bis 5 als Dispersions- bzw. Suspensionsstabilisator bei der Herstellung von Kosmetika und von technischen Produkten, die sich sehr rasch dispergieren sollen.

11. Verwendung des Polysaccharidprodukts nach einem der Ansprüche 1 bis 5 als Dispersions- oder Suspensionsstabilisator bei der Herstellung von flüssigen und halbfesten Zubereitungen wie Sirupen, Lotionen, Säften, Gelen, Hydrosolen und Hydrogelen, Salbenzubereitungen und Pasten.
